# EUROPEAN PATENT APPLICATION

(11) **EP 4 736 844 A1**
(43) Date of publication of application: **06.05.2026**
(21) Application number: 24211013.8
(22) Date of filing: 05.11.2024
(51) Int. Cl.: A61K 9/16, A61K 9/19, A61K 38/26

(54) **PHARMACEUTICAL COMPOSITION COMPRISING A GLP-1 ANALOG AND AN INORGANIC MESOPOROUS MATERIAL**

(71) Applicant: Merck Patent GmbH, 64293 Darmstadt (DE)
(72) Inventor: The designation of the inventor has not yet been filed
(74) Representative: Merck Patent Association

(57) **Abstract**

The present invention relates to pharmaceutical compositions comprising a therapeutic peptide having a molecular weight between 1 kDa and 100 kDa dispersed in an inorganic mesoporous material, said inorganic mesoporous material being in particular mesoporous silica.

## Description

The oral administration of therapeutic peptides presents significant challenges due to their inherent biochemical properties. Peptides, including glucagon-like peptide-1 (GLP-1) analogs such as Exenatide (Byetta, Bydureon), Liraglutide (Victoza, Saxenda), Dulaglutide (Trulicity), Semaglutide (Ozempic, Rybelsus, Wegovy), Lixisenatide (Adlyxin), Albiglutide (Tanzeum), Tirzepatide and Efpeglenatide, are increasingly used in the treatment of type 2 diabetes and obesity. However, their effectiveness and patient compliance are often hindered by the limitations associated with their oral delivery.

One of the primary challenges with the oral administration of peptides is their susceptibility to enzymatic degradation in the gastrointestinal (GI) tract. The GI tract is rich in proteolytic enzymes that rapidly degrade peptides, rendering them inactive before they can exert their therapeutic effects. In addition, the stability of peptides in the acidic environment of the stomach is a critical concern. The acidic pH can lead to the denaturation and degradation of peptide molecules. Formulation strategies, such as enteric coatings and pH-sensitive delivery systems, have been investigated to protect peptides from the harsh gastric environment, but achieving consistent and reliable protection remains a challenge.

Another significant issue is indeed the poor absorption of peptides across the intestinal epithelium. Peptides are large, hydrophilic molecules that do not easily cross the lipid-rich cell membranes of the intestinal lining. This results in low bioavailability when peptides are administered orally. Various approaches, such as the use of absorption enhancers, mucoadhesive agents, and nanoparticle carriers, have been explored to improve the intestinal absorption of peptides, but these strategies often come with their own set of challenges and limitations.

GLP-1 analogs, in particular, exemplify these challenges. These molecules are used extensively in the management of type 2 diabetes and obesity due to their ability to enhance insulin secretion, inhibit glucagon release, slow gastric emptying, and promote satiety. However, their therapeutic potential is often limited by the need for frequent injections, which can lead to poor patient compliance and reduced quality of life. The development of an effective oral delivery system for GLP-1 analogs would represent a significant advancement in the treatment of these conditions, offering a more convenient and patient-friendly administration route.

To date however, only Semaglutide is approved as an oral formulation (Rybelsus^{®}) that is tailored for gastrointestinal absorption to ensure bioavailability.

This necessitates the development of strategies to protect peptides from enzymatic degradation while at the same time ensuring their bioavailability.

It is an object of the present invention to address these issues by providing a pharmaceutical formulation that protects therapeutic peptides, in particular GLP-1 analogs, and enhances their bioavailability, thereby improving patient compliance and treatment outcomes.

The present invention concerns in a first aspect, a pharmaceutical composition comprising a therapeutic peptide having a molecular weight between 1 kDa and 100 kDa dispersed in an inorganic mesoporous material, said inorganic mesoporous material being in particular mesoporous silica.

The present invention also concerns in a second aspect, a pharmaceutical composition comprising a therapeutic peptide having a molecular weight between 1 kDa and 100 kDa in particular a GLP-1 analog, dispersed in an inorganic mesoporous material, said inorganic mesoporous material being in particular mesoporous silica, for use as a drug, in particular in the treatment of type 2 diabetes or obesity.

It also concerns the use of a therapeutic peptide having a molecular weight between 1 kDa and 100 kDa, in particular a GLP-1 analog, dispersed an inorganic mesoporous material, said inorganic mesoporous material being in particular mesoporous silica, for the manufacture of a medicament, said medicament being intended for the treatment of type 2 diabetes or obesity. It further concerns a method for treating a condition, in particular type 2 diabetes or obesity, wherein a pharmaceutical composition comprising a therapeutic peptide having a molecular weight between 1 kDa and 100 kDa, in particular a GLP-1 analog dispersed in an inorganic mesoporous material, said inorganic mesoporous material being in particular mesoporous silica, is administered to a subject in need thereof.

The present invention also concerns in a third aspect the use of an inorganic mesoporous material, in particular mesoporous silica, for preventing the degradation of a therapeutic peptide having a molecular weight between 1 kDa and 100 kDa, in particular a GLP-1 analog, in gastric fluid and/or for preventing the release of a therapeutic peptide having a molecular weight between 1 kDa and 100 kDa, in particular a GLP-1 analog, in gastric fluid and/or for enhancing the bioavailability of a therapeutic peptide having a molecular weight between 1 kDa and 100 kDa, in particular a GLP-1 analog, and/or for delaying the release of a therapeutic peptide having a molecular weight between 1 kDa and 100 kDa, in particular a GLP-1 analog, in the intestine and/or for the targeted delivery of therapeutic peptide having a molecular weight between 1 kDa and 100 kDa, in particular a GLP-1 analog, in the intestine.

The present invention further concerns in a fourth aspect a process for preparing a pharmaceutical composition comprising a therapeutic peptide having a molecular weight between 1 kDa and 100 kDa, in particular a GLP-1 analog, dispersed in an inorganic mesoporous material, said inorganic mesoporous material being in particular mesoporous silica and the pharmaceutical composition obtained by said process.

In the present invention, a therapeutic peptide having a molecular weight between 1 kDa and 100 kDa, designates a chain of amino acids, typically ranging from about 10 to 1000 residues, useful for the treatment of diseases. These peptides can function by interacting with specific receptors, enzymes, or other proteins to modulate physiological pathways. Therapeutic peptides may be used in various medical applications, including hormone replacement, antimicrobial treatments, cancer therapy, and metabolic disorders. Examples are insulin, octreotide, lanreotide, goserelin, leuprolide, bivalirudin, and GLP-1 analogs.

In the present invention, a GLP-1 analog designates a synthetic version of the naturally occurring GLP-1 (glucagon-like peptide-1) hormone, designed to mimic the effects of GLP-1 hormone in the body. Such GLP-1 analogs are peptides used primarily in the treatment of type 2 diabetes and, in some cases, for weight management (obesity). GLP-1 analogs include Exenatide (Byetta^{®}, Bydureon^{®}), Liraglutide (Victoza^{®}, Saxenda^{®}), Dulaglutide (Trulicity^{®}), Semaglutide (Ozempic^{®}, Rybelsus^{®}, Wegovy^{®}), Lixisenatide (Adlyxin^{®}), Albiglutide (Tanzeum^{®}), Tirzepatide and Efpeglenatide.

As used herein, the term "specific surface area" (SSA) is a property of solids defined as the total surface area of a material per unit of mass [m²/g]. According to the invention, the SSA is measured according to DIN ISO 9277:2014-01 by using a "3 Flex Version 3.01-Serial number 324" from Micromeritics Instrument Cooperation, US. The sample is heated up to 250 °C under vacuum. A multi-point measurement is performed with N₂ as carrier gas.

As used herein, the term "non-ordered" refers to materials that lack a regular, repeating structure at the molecular or microscopic level. Unlike ordered inorganic materials which have a well-defined pore structure, non-ordered silica has a more random arrangement of its pores.

As used herein, the term "average pore size" is a property of porous solids defined as the distance between two opposite walls of the pore (diameter of cylindrical pores). According to the invention the average pore size is measured according to ISO 15901-2:2006. Adsorption- and desorption isotherm were measured using N₂ as adsorbent and calculation of pore size and pore volume was done according to Barrett, Joyner and Halenda.

As used herein, the term "D₅₀", "median of a particle size distribution", "average particle size" and "particle size" is a property of solids defined as the diameter in microns where half of the particle population resides above this size, and half resides below this size. The term "median" might refer to half the mass, half the volume, or half the number of particles. For the purposes of this invention, D₅₀ always refers to the volume median, and it is the median for a volume distribution as measured by laser light scattering, using a suitable (wet or dry) method, a suitable obscuration, and settings of the measurement method (e.g. Venturi pressure drop for the dry method) suitable selected so that the measurement is accurate, representative and reproducible. Similarly, 90 percent of volume of the population lies below the D₉₀, and 10 percent of the volume of the population lies below the D₁₀. According to the invention, the particle size is measured in a particle size analyzer LS 13320 Optical Bench (Beckman Coulter, Inc., NJ USA) using the Tornado Dry Powder System (DPS), and implementing a Venturi pressure drop of 10" and where the particle size distribution is determined based on the Fraunhofer model using a sample size of at least 20 ml and 5% obscuration. In the Tornado DPS the sample is placed in a sample holder and delivered to the sensing zone in the optical bench by a vacuum.

In a first aspect, the present invention relates to a pharmaceutical composition comprising a therapeutic peptide having a molecular weight between 1 kDa and 100 kDa, in particular a GLP-1 analog, dispersed in an inorganic mesoporous material.

An inorganic mesoporous material designates a material characterized by its porous structure with average pore size in the range of 2 to 50 nanometers. These materials are composed of inorganic substances such as silica (SiO₂), alumina (Al₂O₃), titania (TiO₂). The term "mesoporous" refers to the specific range of average pore size, distinguishing these materials from microporous (average pore size less than 2 nm) and macroporous (average pore size greater than 50 nm) materials. Mesoporous materials have a high specific surface area due to their well-defined pore structures, making them suitable for adsorption of small molecules and drug delivery. The inorganic nature of these materials often imparts high thermal and chemical stability.

Particularly contemplated inorganic mesoporous materials are mesoporous silica. Examples of mesoporous silica are MCM-41 (having a hexagonal arrangement of cylindrical pores, SBA-15 (having larger pores and thicker walls compared to MCM-41), KIT-6 (having a three-dimensional cubic pore structure), the mesoporous silica sold under the tradename Syloid^{®}, the mesoporous silica sold under the tradename Neusilin^{®}, the mesoporous silica sold under the tradename Aeropearl^{®}, the mesoporous silica sold under the tradename Fujicalin^{®} and the mesoporous silica sold under the tradename Parteck^{®} SLC 500. A preferred mesoporous silica is the silica sold by Merck under the tradename Parteck^{®} SLC 500.

The mesoporous silica has one or more of the following characteristics:
- it is a non-ordered mesoporous silica,
- a particle size distribution D₅₀ from about 5 to 150 µm, preferably from 15 to about 75 µm, in particular from about 17 to 25 µm,
- a particle size distribution D₉₀ from about 30 to about 100 µm, preferably from about 40 to 80 µm, in particular from about 48 to about 54 µm,
- a bulk density from about 0.2 to about 0.8 g/ml, preferably from about 0.3 to about 0.5 g/ml, in particular of about 0.32 g/ml,
- an average pore size (or volume) of from about 2 to about 50 nm, preferably from about 3 to 10 nm, in particular of about 6 nm,
- a specific surface area of at least 300 m²/g, preferably at least 400 m²/g, more preferably of at least 500 m²/g and in particular of about 500 m²/g,
- a total pore volume of at least 0.5 cm³/g, preferably at least 0.7 and in particular of about 0.75 cm³/g.

Preferably, the mesoporous silica has all of these characteristics. More preferably, the mesoporous silica is non-ordered, has a particle size distribution D₅₀ of from about 17 to 25 µm, a bulk density of from about 0.3 to 0.5 g/ml, an average pore size of from about 3 to about 10 nm, a specific surface area of at least about 400 m²/g and a total pore volume of at least 0.7 cm³/g.

In particular, the mesoporous silica is non-ordered, has a particle size distribution D₅₀ of from about 17 to 25 µm, a bulk density of about 0.32 g/ml, an average pore size of from about 6 nm and a specific surface area of about 500 m²/g and a total pore volume of about 0.75 cm³/g.

The therapeutic peptide may have a molecular weight comprised between 1 kDa and 100 kDa, between 1 kDa and 90 kDa, between 1 kDa and 80 kDa, between 1 kDa and 70 kDa, between 1 kDa and 60 kDa, between 1 kDa and 50 kDa, between 1 kDa and 40 kDa, between 1 kDa and 30 kDa, between 1 kDa and 25 kDa, between 1 kDa and 20 kDa, between 1 KDa and 15 KDa, between 1 KDa and 10 KDa, between 1 KDa and 5 KDa or between 2 and 5 kDa.

Particulary contemplated are therapeutic peptides having a molecular weight comprised between between 1 kDa and 40 kDa, between 1 kDa and 20 kDa, between 1 KDa and 15 KDa, between 1 KDa and 10 KDa, between 1 KDa and 5 KDa or between 2 and 5 kDa.

The therapeutic peptide having a molecular weight comprised between 1 kDa and 100 kDa is preferably a GLP-1 analog. Examples of GLP-1 analogs include the ones from the group consisting in Exenatide, Liraglutide, Dulaglutide, Semaglutide, Albiglutide, Tirzepatide and Efpeglenatide, or their mixtures. Preferably the GLP-1 analog is Semaglutide.

The ratio of the therapeutic peptide having a molecular weight between 1 kDa and 100 kDa to the inorganic mesoporous material that is present in the composition may vary widely. Said amount is of course dependent on the intrinsic properties of the inorganic mesoporous material (e.g. its loading capacity) and the intrinsic properties of the therapeutic peptide having a molecular weight between 1 kDa and 100 kDa (e.g. its solubility and molecular weight).

It has been observed that at low (therapeutic peptide having a molecular weight between 1 kDa and 100 kDa):(inorganic mesoporous material) ratio, such as 1:40, the GLP-1 analog is neither released in a medium having a pH of 1.2, mimicking the pH of the stomach nor in a medium having a pH of 6.8, mimicking the pH of the intestine. Hence, (therapeutic peptide having a molecular weight between 1 kDa and 100 kDa):(inorganic mesoporous material) ratio should remain below this threshold, e.g. at a maximum ratio of 1:30.

Typically, the ratio (therapeutic peptide having a molecular weight between 1 kDa and 100 kDa):(inorganic mesoporous material) is between 1:30 and 1:1, preferably between 1:30 and 1:10, more preferably between 1:20 and 1:10, in particular of about 1:20 or 1:10.

In an embodiment, the inorganic mesoporous material is mesoporous silica and the (GLP-1 analog):(mesoporous silica) is between 1:30 and 1:10, preferably between 1:20 and 1:10, in particular of about 1:20 or 1:10.

In another embodiment, the GLP-1 analog is semaglutide and the (GLP-1 analog):(mesoporous silica) is between 1:30 and 1:10, preferably between 1:20 and 1:10, in particular of about 1:20 or 1:10.

In yet another embodiment, the inorganic mesoporous material is a mesoporous silica having a particle size distribution D₅₀ of from about 17 to 25 µm, a bulk density of from about 0.3 to 0.5 g/ml, an average pore size of from about 3 to about 10 nm, a specific surface area of at least about 400 m²/g and a total pore volume of at least 0.7 cm³/g; and the (GLP-1 analog):(mesoporous silica) is between 1:30 and 1:10, preferably between 1:20 and 1:10, in particular of about 1:20 or 1:10. The GLP-1 analog may preferably be one from the group consisting in Exenatide, Liraglutide, Dulaglutide, Semaglutide, Albiglutide, Tirzepatide and Efpeglenatide, in particular semaglutide.

In a further embodiment, the mesoporous silica has a particle size distribution D₅₀ of from about 17 to 25 µm, a bulk density of about 0.32 g/ml, an average pore size of from about 6 nm, a specific surface area of about 500 m²/g and a total pore volume of about 0.75 cm³/g; and the (GLP-1 analog):(mesoporous silica) is between 1:30 and 1:10, preferably between 1:20 and 1:10, in particular of about 1:20 or 1:10. The GLP-1 analog may preferably be one from the group consisting in Exenatide, Liraglutide, Dulaglutide, Semaglutide, Albiglutide, Tirzepatide and Efpeglenatide, in particular semaglutide.

The pharmaceutical composition may comprise one or more further excipients, for example selected from the group consisting in colorants, flavorants, antioxidants, preservatives, surfactants or intestinal permeation enhancers.

Particularly contemplated excipients are intestinal permeation enhancers in particular Salcaprozate sodium (SNAC, Sodium 8-[(2-hydroxybenzoyl)amino]octanoate), bile salts or sodium caprate, more particularly SNAC.

In a second aspect, the present invention concerns a pharmaceutical composition comprising a therapeutic peptide having a molecular weight between 1 kDa and 100 kDa dispersed in an inorganic mesoporous material as defined in the first aspect, for use as a drug, especially in the treatment of type 2 diabetes or obesity.

The pharmaceutical composition may be administered by any route known to those skilled in the art, e.g. orally or parenterally.

As a result of the stabilizing effect of the inorganic mesoporous material on the therapeutic peptide having a molecular weight between 1 kDa and 100 kDa and its ability to prevent its release and degradation in the stomach, the pharmaceutical composition is preferably administered orally. In one embodiment, the present invention therefore concerns a pharmaceutical composition as defined in the first aspect, for use as a drug, especially in the treatment of type 2 diabetes or obesity, wherein the pharmaceutical composition is administered orally.

The pharmaceutical composition may be a tablet (hard, chewable...), an orally disintegrating tablet (effervescent, sublingual...), a capsule (soft gelatin capsule...), a suspension, a syrup, an elixir, a powder (for resuspension), granules, lozenges or troches, a gel or an oral film, in particular a tablet.

As another result of the stabilizing effect of the inorganic mesoporous material on the therapeutic peptide having a molecular weight between 1 kDa and 100 kDa and its ability to prevent its release and degradation in the stomach, the pharmaceutical composition needs not being coated. If desired, a coating may however be applied, for example to slow the release of the therapeutic peptide having a molecular weight between 1 kDa and 100 kDa further.

In this second aspect, the present invention also relates to the use of a therapeutic peptide having a molecular weight between 1 kDa and 100 kDa dispersed an inorganic mesoporous material, for the manufacture of a medicament, said medicament being intended for the treatment of type 2 diabetes or obesity, said medicament being preferably administered orally.

In this second aspect, the present invention also relates to a method for treating a condition, in particular type 2 diabetes or obesity, wherein a pharmaceutical composition comprising a therapeutic peptide having a molecular weight between 1 kDa and 100 kDa dispersed in an inorganic mesoporous material as defined in the first aspect, is administered to a subject in need thereof, preferably orally.

In a third aspect, the present invention relates to the use of an inorganic mesoporous material for one or more of the following purposes:
- improving the bioavailability of a therapeutic peptide having a molecular weight between 1 kDa and 100 kDa,
- preventing the degradation of a therapeutic peptide having a molecular weight between 1 kDa and 100 kDa in gastric fluid,
- preventing the release of a therapeutic peptide having a molecular weight between 1 kDa and 100 kDa in gastric fluid,
- delaying the release of a therapeutic peptide having a molecular weight between 1 kDa and 100 kDa in the intestine,
- the targeted delivery of a therapeutic peptide having a molecular weight between 1 kDa and 100 kDa in the intestine.

In the course of the studies, it has been found that the loading of a therapeutic peptide having a molecular weight between 1 kDa and 100 kDa, such as Semaglutide, onto an inorganic mesoporous material, such as a mesoporous silica having a particle size distribution D₅₀ of from about 17 to 25 µm, a bulk density of about 0.32 g/ml, an average pore size of from about 6 nm and a specific surface area of about 500 m²/g, results in a composition that does not release the therapeutic peptide having a molecular weight between 1 kDa and 100 kDa, such as Semaglutide, in a medium at pH 1.2 (such as the gastric environment) but releases it in a medium at pH 6.8 (such as the intestine environment).

As a result, the therapeutic peptide having a molecular weight between 1 kDa and 100 kDa is protected from degradation in the stomach and is only released in the intestine, where it can be absorbed to produce its therapeutic effect.

According to the invention, "preventing the release of a therapeutic peptide having a molecular weight between 1 kDa and 100 kDa in gastric fluid" means that less than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1% of the therapeutic peptide having a molecular weight between 1 kDa and 100 kDa is released in the stomach. The amount of the therapeutic peptide having a molecular weight between 1 kDa and 100 kDa that is released in the gastric fluid may be determined using the method described in the example of the present application using FASSGF (fasted state simulated gastric fluid) over the course of one hour. Preferably, "preventing the release of a therapeutic peptide having a molecular weight between 1 kDa and 100 kDa in gastric fluid" means that less than 10%, 9%, 8%, 7%, 6%, 5%, 4%, 3%, 2% or 1% of the therapeutic peptide having a molecular weight between 1 kDa and 100 kDa is released in the stomach. According to the invention, "delaying the release of a therapeutic peptide having a molecular weight between 1 kDa and 100 kDa" in the intestine means that less than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1% of the GLP-1 analog is released in the stomach. The amount of the therapeutic peptide having a molecular weight between 1 kDa and 100 kDa that is released in the gastric fluid may be determined using the method described in the example of the present application using FASSGF (fasted state simulated gastric fluid) over the course of one hour. Preferably, "preventing the release of a therapeutic peptide having a molecular weight between 1 kDa and 100 kDa in gastric fluid" means that less than 10%, 9%, 8%, 7%, 6%, 5%, 4%, 3%, 2% or 1% of the therapeutic peptide having a molecular weight between 1 kDa and 100 kDa is released in the stomach.

According to the invention, "the targeted delivery of a therapeutic peptide having a molecular weight between 1 kDa and 100 kDa in the intestine" means that less than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1% of the therapeutic peptide having a molecular weight between 1 kDa and 100 kDa is released in the stomach. The amount of the therapeutic peptide having a molecular weight between 1 kDa and 100 kDa that is released in the gastric fluid may be determined using the method described in the example of the present application using FASSGF (fasted state simulated gastric fluid) over the course of one hour. Preferably, "the targeted delivery of a therapeutic peptide having a molecular weight between 1 kDa and 100 kDa in the intestine" means that less than 10%, 9%, 8%, 7%, 6%, 5%, 4%, 3%, 2% or 1% of the therapeutic peptide having a molecular weight between 1 kDa and 100 kDa is released in the stomach.

In this aspect of the invention, the inorganic mesoporous material is preferably a mesoporous silica.

The mesoporous silica has one or more of the following characteristics:
- it is non-ordered,
- a particle size distribution D₅₀ from about 5 to 150 µm, preferably from 15 to about 75 µm, in particular from about 17 to 25 µm,
- a particle size distribution D₉₀ from about 30 to about 100 µm, preferably from about 40 to 80 µm, in particular from about 48 to about 54 µm,
- a bulk density from about 0.2 to about 0.8 g/ml, preferably from about 0.3 to about 0.5 g/ml, in particular of about 0.32 g/ml,
- an average pore size (or volume) of from about 2 to about 50 nm, preferably from about 3 to 10 nm, more preferably from about 6 nm,
- a specific surface area of at least 300 m²/g, preferably at least 400 m²/g, more preferably of at least 500 m²/g and even more preferably of about 500 m²/g.
- a total pore volume of at least 0.5 cm³/g, preferably at least 0.7 and in particular of about 0.75 cm³/g.

Preferably, the mesoporous silica has all of these characteristics. More preferably, the mesoporous silica is non-ordered, has a particle size distribution D₅₀ of from about 17 to 25 µm, a bulk density of from about 0.3 to 0.5 g/ml, an average pore size of from about 3 to about 10 nm, a specific surface area of at least about 400 m²/g and a total pore volume of at least 0.7 cm³/g.

In particular, the mesoporous silica is non-ordered, has a particle size distribution D₅₀ of from about 17 to 25 µm, a bulk density of about 0.32 g/ml, an average pore size of from about 6 nm and a specific surface area of about 500 m²/g and a total pore volume of about 0.75 cm³/g.The therapeutic peptide may have a molecular weight comprised between 1 kDa and 100 kDa, between 1 kDa and 90 kDa, between 1 kDa and 80 kDa, between 1 kDa and 70 kDa, between 1 kDa and 60 kDa, between 1 kDa and 50 kDa, between 1 kDa and 40 kDa, between 1 kDa and 30 kDa, between 1 kDa and 25 kDa, between 1 kDa and 20 kDa, between 1 KDa and 15 KDa, between 1 KDa and 10 KDa, between 1 KDa and 5 KDa or between 2 and 5 kDa.

Particulary contemplated are therapeutic peptides having a molecular weight comprised between between 1 kDa and 40 kDa, between 1 kDa and 20 kDa, between 1 KDa and 15 KDa, between 1 KDa and 10 KDa, between 1 KDa and 5 KDa or between 2 and 5 kDa.

The therapeutic peptide having a molecular weight comprised between 1 kDa and 100 kDa is preferably a GLP-1 analog. Examples of GLP-1 analogs include the ones from the group consisting in Exenatide, Liraglutide, Dulaglutide, Semaglutide, Albiglutide, Tirzepatide and Efpeglenatide, or their mixtures. Preferably the GLP-1 analog is Semaglutide.

Typically, the ratio (therapeutic peptide having a molecular weight between 1 kDa and 100 kDa):(inorganic mesoporous material) is between 1:30 and 1:1, preferably between 1:30 and 1:10, more preferably between 1:20 and 1:10, in particular of about 1:20 or 1:10.

In an embodiment, the inorganic mesoporous material is mesoporous silica and the (GLP-1 analog):(mesoporous silica) is between 1:30 and 1:10, preferably between 1:20 and 1:10, in particular of about 1:20 or 1:10.

In another embodiment, the GLP-1 analog is semaglutide and the (GLP-1 analog):(mesoporous silica) is between 1:30 and 1:10, preferably between 1:20 and 1:10, in particular of about 1:20 or 1:10.

In yet another embodiment, the inorganic mesoporous material is a mesoporous silica having a particle size distribution D₅₀ of from about 17 to 25 µm, a bulk density of from about 0.3 to 0.5 g/ml, an average pore size of from about 3 to about 10 nm, a specific surface area of at least about 400 m²/g and a total pore volume of at least 0.7 cm³/g; and the (GLP-1 analog):(mesoporous silica) is between 1:30 and 1:10, preferably between 1:20 and 1:10, in particular of about 1:20 or 1:10. The GLP-1 analog may preferably be one from the group consisting in Exenatide, Liraglutide, Dulaglutide, Semaglutide, Albiglutide, Tirzepatide and Efpeglenatide, in particular semaglutide.

In a further embodiment, the mesoporous silica has a particle size distribution D₅₀ of from about 17 to 25 µm, a bulk density of about 0.32 g/ml, an average pore size of from about 6 nm, a specific surface area of about 500 m²/g and a total pore volume of about 0.75 cm³/g; and the (GLP-1 analog):(mesoporous silica) is between 1:30 and 1:10, preferably between 1:20 and 1:10, in particular of about 1:20 or 1:10. The GLP-1 analog may preferably be one from the group consisting in Exenatide, Liraglutide, Dulaglutide, Semaglutide, Albiglutide, Tirzepatide and Efpeglenatide, in particular semaglutide.

In a fourth aspect, the invention relates to a process for preparing a pharmaceutical composition comprising a therapeutic peptide having a molecular weight between 1 kDa and 100 kDa dispersed in an inorganic mesoporous material, said inorganic mesoporous material being in particular mesoporous silica and the pharmaceutical composition obtained by said process.

The process comprises the steps of:
a. contacting an inorganic mesoporous material with a therapeutic peptide having a molecular weight between 1 kDa and 100 kDa in a liquid medium, to obtain a suspension,
b. drying the suspension to obtain the pharmaceutical composition comprising the therapeutic peptide having a molecular weight between 1 kDa and 100 kDa dispersed in the inorganic mesoporous material.

Step a. is conventionally performed by agitating a mixture of the inorganic mesoporous material and the therapeutic peptide having a molecular weight between 1 kDa and 100 kDa in the liquid medium. Agitation may be performed by stirring (manual or mechanical), shaking (manual, mechanical or vortexing), homogenization (high-pressure homogenizers, ultrasonic homogenizers), sonication, fluid mixing (static mixers, dynamic mixers), spray mixing or turbulence.

The liquid medium may be any solvent in which the therapeutic peptide having a molecular weight between 1 kDa and 100 kDa is soluble or suspended. Preferably, the liquid medium is a buffered medium, such as phosphate buffered saline.

In one embodiment, step a. is a continuous impregnation method, in particular as described in PCT application WO2022/073621.

The present invention therefore concerns, in one embodiment, a process for preparing a pharmaceutical composition comprising a therapeutic peptide having a molecular weight between 1 kDa and 100 kDa dispersed in an inorganic mesoporous material, comprising the steps of:
a1.continuously dispensing a drug solution or a drug suspension containing at least one API and feeding a porous carrier in a controlled ratio into a continuous impregnation device,
a2.continuously mixing and conveying the drug solution or drug suspension and the porous carrier in the continuous impregnation device to obtain a mixed suspension,
b. drying the mixed suspension to obtain the pharmaceutical composition comprising the GLP-1 analog dispersed in the inorganic mesoporous material.

Step b. of drying of the suspension may be performed by any means known to those skilled in the art, such as spray drying, freeze drying (lyophilization), rotary evaporation, vacuum drying, tray drying, fluidized bed drying, drum drying, microwave drying, infrared drying, air drying.

Drying is preferably performed, and in some cases repeated until the mass variation of the pharmaceutical composition is less than 1% before and after drying.

### Description of the Figures

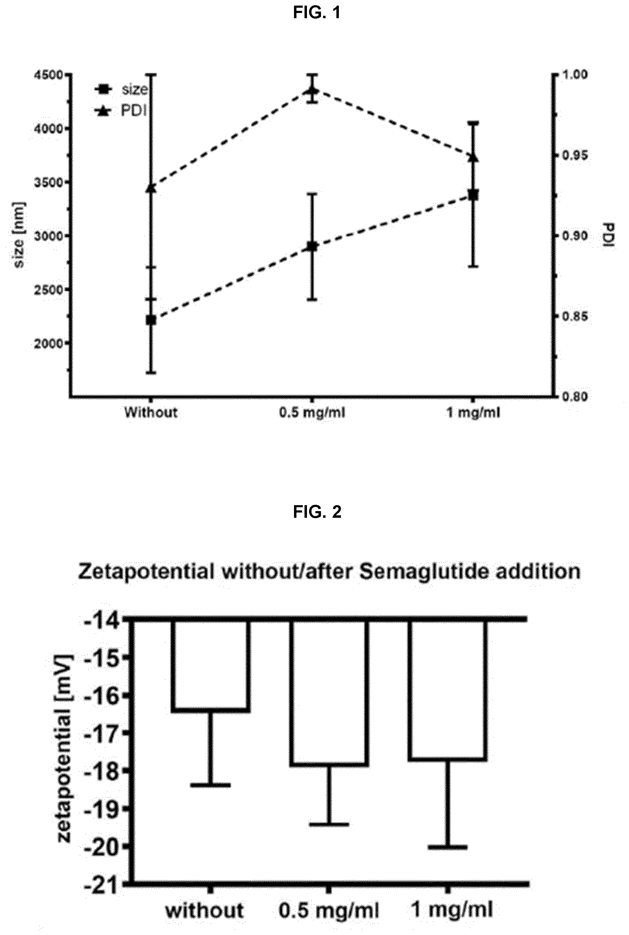

**Figure 1** represents the size (Z-average) and size distribution, described by the polydispersity index (PDI), of liposomes as determined by photon correlation spectroscopy.
**Figure 2** represents the zeta potential as measured using the Zetasizer Nano ZS.

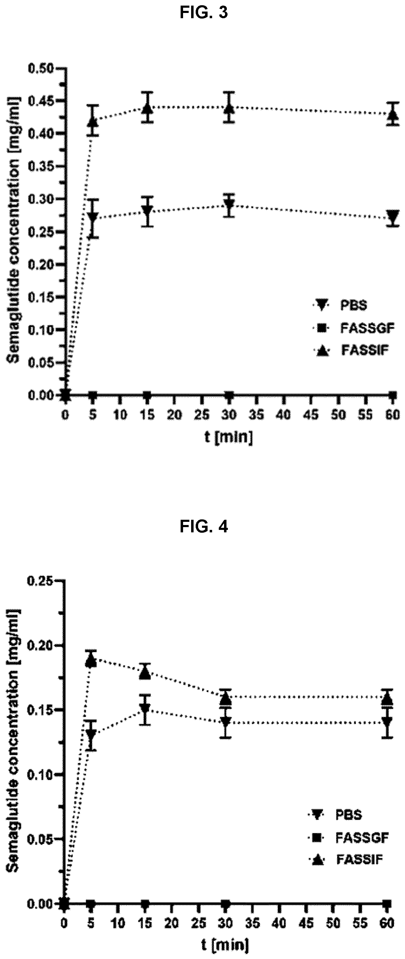

**Figure 3** represents the release of semaglutide from a formulation containing Parteck^{®} SLC and Semaglutide in concentration of 10 mg SLC + 1 mg Semaglutide in three different media: phosphate buffer pH 6.8, FASSGF (fasted state simulated gastric fluid) and FASSIF (fasted state simulated intestinal fluid) at 37 °C over a time period of 60 minutes.
**Figure 4** represents the release of semaglutide from a formulation containing Parteck^{®} SLC and Semaglutide in concentration of 10 mg SLC + 0.5 mg Semaglutide in three different media: phosphate buffer pH 6.8, FASSGF (fasted state simulated gastric fluid) and FASSIF (fasted state simulated intestinal fluid) at 37 °C over a time period of 60 minutes.

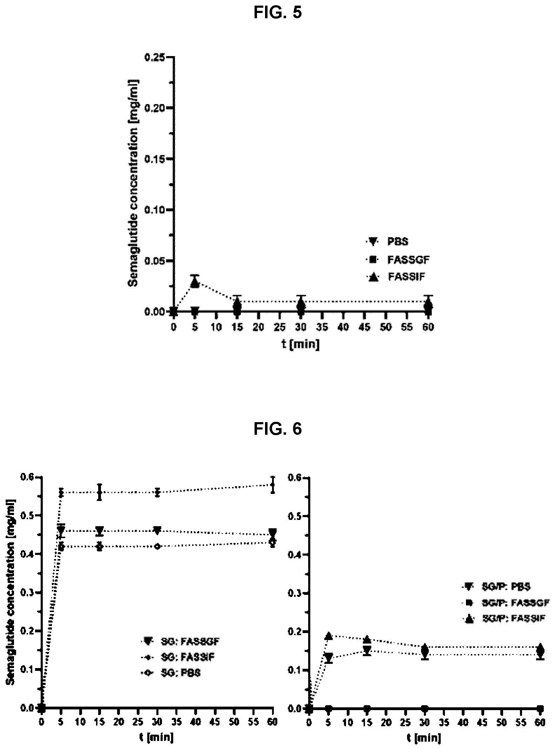

**Figure 5** represents the release of semaglutide from a formulation containing Parteck^{®} SLC and Semaglutide in concentration of 20 mg SLC + 0.5 mg Semaglutide in three different media: phosphate buffer pH 6.8, FASSGF (fasted state simulated gastric fluid) and FASSIF (fasted state simulated intestinal fluid) at 37 °C over a time period of 60 minutes.
**Figure 6** represents the release of free semaglutide (left graph) and the release of semaglutide from a formulation containing Parteck^{®} SLC and Semaglutide in concentration of 20 mg SLC + 0.5 mg Semaglutide (right graph) in three different media: phosphate buffer pH 6.8, FASSGF (fasted state simulated gastric fluid) and FASSIF (fasted state simulated intestinal fluid) at 37 °C over a time period of 60 minutes.

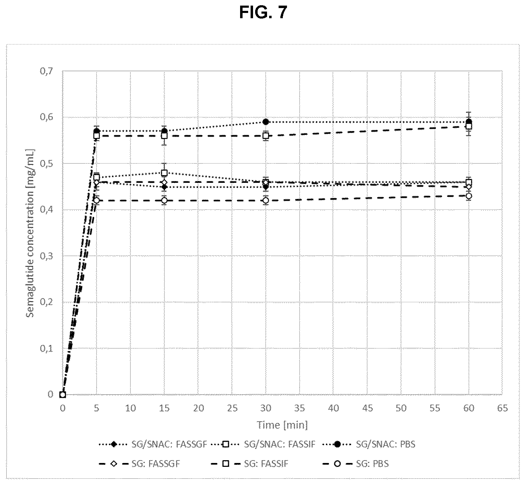

**Figure 7** represents the release of semaglutide from a formulation containing semaglutide and SNAC and the release of semaglutide from a formulation containing Parteck^{®} SLC and Semaglutide in concentration of 20 mg SLC + 0.5 mg Semaglutide (right graph) in three different media: phosphate buffer pH 6.8, FASSGF (fasted state simulated gastric fluid) and FASSIF (fasted state simulated intestinal fluid) at 37 °C over a time period of 60 minutes.

### Examples:

### Preparation of Parteck^{®} SLC 500 - semaglutide formulations and freeze-drying

Parteck^{®} SLC 500 is a mesoporous silica having a particle size distribution D₅₀ of from about 17 to 25 µm, a bulk density of about 0.32 g/ml, an average pore size of from about 6 nm, a specific surface area of about 500 m²/g and a total pore volume of about 0.7 cm³/g.

Parteck^{®} SLC 500 and semaglutide were weighted in Eppendorf tubes in required amounts and subsequently suspended in PBS. After vortexing for 60 s, the suspension were freeze-dryied using the freeze dryer Epsilon 2-4 LSCplus (Martin Christ Gefriertrocknungsanlagen GmbH, Osterode am Harz, Germany), and the process was run as follows: (I) Freezing stage: The samples were cooled down to -40 °C for 2 h and held at this temperature for 4 h. (II) Primary drying stage: The vacuum was drawn to 0.1 mbar over 1 h and held for 24 h. (III) Secondary drying stage: The vacuum was drawn to 0.01 mbar over 2 h, and the temperature was steadily increased from -40 °C to 0 °C. The settings were then held for 40 h for the secondary drying stage.

### Example 1 - Characterisation of Parteck^{®} SLC 500 - semaglutide suspension

The size (Z-average) and size distribution, described by the polydispersity index (PDI), of liposomes were determined by photon correlation spectroscopy.

The suspension was diluted 1:20 in phosphate buffered saline (PBS) in a single-use polystyrene cuvette and measured with Zetasizer Nano ZS (Malvern Panalytical, Malvern, UK). The default settings of the automatic mode of the Zetasizer are shown in Table 1.

The zeta potential was measured using the Zetasizer Nano ZS. Liposomes were diluted 1:20 in buffer (50 mM potassium dihydrogen phosphate, pH 7.4). An equilibration time of 60 seconds (s) was set, and three measurements with 20 runs were performed.

**Table 1**

| **Parameter** | **Setting** |
|---|---|
| Number of measurements | 3 |
| Run duration | 10 s |
| Number of runs | 10 |
| Equilibration time | 60 s |
| Temperature | 25 °C |
| Dielectric constant | 78.5 F/m |
| Backscattering mode | 173 °C |
| Voltage selection | Automatic |
| Equation | Smoluchowski equation |

The characteristics of Parteck^{®} SLC 500 with and without semaglutide are shown in Figure 1. While the size increases with increasing concentrations of semaglutide, no significant difference in zetapotential is observed.

### Example 3 - Dissolution studies

### RP-HPLC method for quantification of semaglutide

Semaglutide concentration in samples was determined using HPLC (Agilent 1100 Series, Agilent, Santa Clara, United States) with a C18 column (Chromolith^{®} Performance RP-18e, 100-3 mm, Merck KGaA, Darmstadt, Germany) applying a linear gradient of 0.1% TFA in water (eluent A) to 0.1% TFA in acetonitrile (eluent B) within 5 min (flow rate 2 ml/min; UV absorbance λ = 214 nm). To prepare a stock solution with a concentration of 2 mg/ml, 1 mg of semaglutide was dissolved in 250 µl of 0.1% TFA in water, then the pH was adjusted to pH 7.0 upon dilution with 250 µl of Citrate-Phosphat-Buffer (CPB, pH 7.0, 10 0mM). Serial dilutions were carried out with HPLC-grade water starting from 2 mg/ml to reach concentrations of 1.5 mg/ml, 1 mg/ml, 0.75 mg/ml, 0.5 mg/ml, 0.25 mg/ml, 0.1 mg/ml and 0.01 mg/ml. The established HPLC method for quantification of semaglutide revealed robust values (r2>0.99).

The release of 1 mg Semaglutide from the formulations containing Parteck^{®} SLC and Semaglutide in concentrations of 10 mg SLC + 1 mg Semaglutide, 10 mg SLC + 0.5 mg Semaglutide and 20 mg SLC and 0.5 mg of Semaglutide were subjected to 1 ml of phosphate buffer pH 6.8, FASSGF (fasted state simulated gastric fluid) and FASSIF (fasted state simulated intestinal fluid) at 37 °C. The concentration of free drug was determined over a time period of 60 minutes with sampling points of 50 µl at 5, 15, 30 and 60 minutes.

The sample was centrifuged to allow the SLC to be separated. The free Semaglutide concentration was determined via RP-HPLC as described above.

The release of semaglutide out of the Parteck^{®} SLC 500 formulations was investigated using different amounts of semaglutide and Parteck^{®} SLC 500.

The results of a formulation containing 10 mg Parteck^{®} SLC 500 and 1 mg of semaglutide are shown in Figure 3. While no release in FASSGF is visible, delayed release in FASSGF can be observed (max. 44% of total semaglutide).

The results of a formulation containing 10 mg Parteck^{®} SLC 500 and 0.5 mg of semaglutide are shown in Figure 4. Using a combination of 10 mg Parteck^{®} SLC 500 and 0.5 mg of semaglutide showed the similar behavior in FASSGF as the formulation containing 10 mg Parteck^{®} SLC 500 and 1 mg of semaglutide. In FASSIF the release was max. 38% of total semaglutide.

The results of a formulation containing 20 mg Parteck^{®} SLC 500 and 0.5 mg of semaglutide are shown in Figure 5. While no release in FASSGF was visible, increasing the amount of Parteck^{®} SLC 500 (20 mg Parteck^{®} SLC 500 and 0.5 mg of semaglutide) prohibited also the release of semaglutide in FASSIF (max. 3% of total semaglutide).

For the formulation containing 10 mg Parteck^{®} SLC 500 and 0.5 mg of semaglutide formulation, the release behaviour was compared to free semaglutide (Figure 6). This study showed a complete release of semaglutide within 5 minutes.

For the formulation containing 10 mg Parteck^{®} SLC 500 and 0.5 mg of semaglutide formulation, the release behaviour was compared to a combination of semaglutide and SNAC (Figure 7). This study showed a complete release of semaglutide within 5 minutes.

To obtain a fluorescent-labelled conjugate, semaglutide was coupled to ATTO-488 (ATTO-TEC, Siegen, Germany). 2 mg of the compound was dissolved in PBS (adjusted to pH 9.0) (Sigma-Aldrich, Taufkirchen, Germany). The solution was then incubated with 2 equivalents of ATTO-488-NHS-Ester (dissolved in dimethyl sulfoxide (DMSO)) for up to 12 hours. The conjugates were purified using preparative reversed-phase HPLC (331 series, Gilson Inc., Middleton, WI, USA; Reprosil-Pur 120 C-18-AQ column, 5 µm, 250 × 25 mm; Dr. Maisch HPLC GmbH, Ammerbuch, Germany) with a linear gradient with water (+0.1% trifluoroacetic acid [TFA]) and acetonitrile (+0.1% TFA). To verify the identity of the conjugates, their mass was determined by LC/MS (Exactive Plus Orbitrap, Thermo Fisher Scientific Inc., Waltham, MA, USA).

### Permeability assay

CaCo-2 were cultivated in Dulbecco's modified Eagle's medium (DMEM) with 4,5 g/L D-glucose supplemented with 10 % fetal bovine serum, 1 % of non-essential amino acids, sodium pyruvate and penicillin/streptomycin (10 000 U/mL penicillin, 10 mg/mL streptomycin in 0.9 % saline solution). Cells were collected at 80 % confluency and seeded on ThinCerts^{®} (0.336 cm2, 3 µm pore size, PET) at a density of 20 000 cells per well. ThinCerts^{®} were maintained in 200 µL and 850 µL media in the apical and basal chamber. The cells were grown for 26 days with media being completely changed after the first 24 h and then every 2-3 days in both compartments. Trans-epithelial electrical resistance (TEER) was determined at day 21 and day of experiment (day 26) using a Millicell^{®} ERS (Merck KGaA, Darmstadt, Germany) measuring three times per ThinCerts^{®} to check for a TEER value of at least 300 Ω·cm2. On the day of experiment, Hank's balanced salt solution (HBSS) was supplemented with HEPES to a final concentration of 25 mM. Media was removed and each cell layer was washed two times with HBSS/HEPES and transferred to a new 24-well plate containing 850 µL HBSS/HEPES. After equilibration for 15 min at assay conditions (37 °C, 150 rpm), apical HBSS/HEPES was removed and the Atto-labelled samples diluted in HBSS/HEPES were applied. Samples of 50 µL volume were drawn from the basal chamber immediately as well as after 30, 90, 120, and 180 min and replaced by fresh HBSS/HEPES. After 240 min, the final samples of 50 µL were drawn from both chambers. The TEER-values were determined again, as described before. All measurements were performed in triplicate. The samples were transferred to a black 96-well plate and measured at a wavelength of 485 nm excitation and 535 nm emission. Final concentrations were calculated via calibration curve using excel 2021

The formulation containing 10 mg Parteck^{®} SLC 500 and 1 mg of semaglutide was tested for *in vitro* uptake on Caco-2 cells (Transwell studies) in comparison to semaglutide / SNAC and free semaglutide. The results showed that the uptake of semaglutide in combination with Parteck^{®} SLC is increased in comparison to the free drug and reaches at least the same improvement factor as the combination of semaglutide with SNAC (Table 2).

**Table 2. Improvement of formulations in comparison to free semaglutide on Caco-2 cells (n=3)**

| **Timepoint [h]** | **Improvement of 0.5 mg semaglutide/10 mg of Parteck^{®} SLC to free semaglutide** | **Improvement of 0.5 mg semaglutide/15 mg of SNAC to free semaglutide** |
|---|---|---|
| 0.5 | 1.3 | 0.9 |
| 1 | 1.6 | 1.4 |
| 2 | 1.6 | 0.6 |
| 3 | 1.2 | 1.1 |
| 4 | 0.9 | 0.6 |

Taken together, these results provide evidence that the combination of semaglutide and an inorganic, mesoporous material such as mesoporous silica results in an enhancement of the bioavailability of GLP-1 analogs such as semaglutide, prevent their degradation in gastric fluid, prevent their release in gastric fluid and allow for their targeted delivery in the intestine.

## Claims

1. A pharmaceutical composition comprising a therapeutic peptide having a molecular weight between 1 kDa and 100 kDa, dispersed in an inorganic, mesoporous material.

2. The pharmaceutical composition according to claim 1, wherein the inorganic mesoporous material is mesoporous silica.

3. The pharmaceutical composition according to claim 2, wherein the mesoporous silica is non-ordered and/or has a particle size distribution D₅₀ of about 17 to 25 µm and/or average pore size of about 6 nm and/or a specific surface of more than 400 m²/g, in particular of about 500 m²/g, and/or a total pore volume of about 75 cm³/g, in particular of about 0.75 cm³/g.

4. The pharmaceutical composition according to any one of the preceding claims, wherein the therapeutic peptide is a GLP-1 analog, preferably selected from the group consisting in Exenatide, Liraglutide, Dulaglutide, Semaglutide, Albiglutide, Tirzepatide and Efpeglenatide

5. The pharmaceutical composition according to any one of the preceding claims, wherein the GLP-1 analog is Semaglutide.

6. The pharmaceutical composition according to any of the preceding claims, wherein the ratio of the therapeutic peptide having a molecular weight between 1 kDa and 100 kDa to the inorganic mesoporous material is between 1:30 and 1:1, preferably between 1:30 and 1:10, more preferably between 1:20 and 1:10, in particular of about 1:20 or 1:10.

7. The pharmaceutical composition according to any one of the preceding claims, wherein the pharmaceutical composition further comprises one or more excipients selected from the group consisting in colorants, flavorants, antioxidants, preservatives, surfactants or intestinal permeation enhancers, preferably an intestinal permeation enhancer that is salcaprozate sodium (SNAC).

8. A pharmaceutical composition according to any of the preceding claims, for use as a drug, in particular in the treatment of a condition selected from the group consisting in type 2 diabetes and obesity.

9. The pharmaceutical composition for use according to the immediately preceding claim, wherein the pharmaceutical composition is administered orally.

10. Use of an inorganic mesoporous material for one or more of the following:
• improving the bioavailability of a therapeutic peptide having a molecular weight between 1 kDa and 100 kDa,
• preventing the degradation of a therapeutic peptide having a molecular weight between 1 kDa and 100 kDa in gastric fluid,
• preventing the release of a therapeutic peptide having a molecular weight between 1 kDa and 100 kDa in gastric fluid,
• the targeted delivery of a therapeutic peptide having a molecular weight between 1 kDa and 100 kDa in the intestine,
wherein the therapeutic peptide having a molecular weight between 1 kDa and 100 kDa is dispersed in said inorganic mesoporous material.

11. The use according to claim 10, wherein the inorganic mesoporous material is mesoporous silica.

12. The use according to claim 11, wherein the mesoporous silica is non-ordered and/or has a particle size distribution D₅₀ of about 17 to 25 µm and/or average pore size of about 6 nm and/or a specific surface of more than 400 m²/g, in particular of about 500 m²/g, and/or a total pore volume of about 75 cm³/g, in particular of about 0.75 cm³/g.

13. The use according to any one of claims 10 to 12, wherein the therapeutic peptide is a GLP-1 analog, preferably selected from the group consisting in Exenatide, Liraglutide, Dulaglutide, Semaglutide, Albiglutide, Tirzepatide and Efpeglenatide, preferably semaglutide.

14. The pharmaceutical composition according to any of the preceding claims, wherein the ratio of the therapeutic peptide having a molecular weight between 1 kDa and 100 kDa to the inorganic mesoporous material is between 1:30 and 1:1, preferably between 1:30 and 1:10, more preferably between 1:20 and 1:10, in particular of about 1:20 or 1:10.

15. A process for preparing a pharmaceutical composition according to any one of claims 1 to 7, comprising a therapeutic peptide having a molecular weight between 1 kDa and 100 kDa dispersed in an inorganic mesoporous material, comprising the steps of:
a. contacting an inorganic mesoporous material with a therapeutic peptide having a molecular weight between 1 kDa and 100 kDa in a liquid medium, to obtain a suspension,
b. drying the suspension to obtain the pharmaceutical composition comprising the therapeutic peptide having a molecular weight between 1 kDa and 100 kDa dispersed in an inorganic mesoporous material.
